# FASCICULE DE BREVET EUROPEEN

(11) **EP 3 528 947 B1**
(45) Date de publication et mention de la délivrance du brevet: **22.07.2020**
(21) Numéro de dépôt: 17797695.8
(22) Date de dépôt: 23.10.2017
(51) Int. Cl.: A61B 10/02, G01N 1/10, B01L 3/00, A61B 10/00, G01N 1/02

(54) **DISPOSITIF DE PRÉLÈVEMENT D'UN ÉCHANTILLON ET SYSTÈME D'ANALYSE D'UN ÉCHANTILLON COMPRENANT UN TEL DISPOSITIF**
VORRICHTUNG ZUR ENTNAHME EINER PROBE UND PROBENANALYSESYSTEM MIT SOLCH EINER VORRICHTUNG
DEVICE FOR TAKING A SAMPLE AND SAMPLE ANALYSIS SYSTEM COMPRISING SUCH A DEVICE

(30) Priorité: 24.10.2016 FR 1660289
(43) Date de publication de la demande: 28.08.2019
(73) Titulaire: Commissariat à l'énergie atomique et aux énergies alternatives, 75015 Paris (FR)
(72) Inventeur: VOLLAND, Hervé, 91400 Orsay (FR)
(74) Mandataire: Brevalex
(86) Numéro de dépôt international: PCT/FR2017/052913
(87) Numéro de publication internationale: WO 2018/078266

(56) Documents cités:
- US-A- 4 180 383
- US-A- 4 580 577
- US-A- 5 211 182
- US-A- 5 352 410
- US-A1- 2005 180 882
- US-A1- 2010 089 181

## Description

### DOMAINE TECHNIQUE

La présente invention appartient au domaine technique des dispositifs pour collecter et transporter des échantillons et notamment des échantillons liquides ou surfaciques.

En effet, la présente invention propose un dispositif à trois modules connectables les uns aux autres de façon mécanique et/ou fluidique grâce auxquels l'échantillon est prélevé, traité et conservé avec un minimum de manipulations de l'utilisateur. Avantageusement ces manipulations se résument à un prélèvement tel qu'un pipetage ou un frottement suivi d'opérations simples telles que vissage, clipsage ou emboutissage. Une fois l'échantillon prélevé, le dispositif selon l'invention permet, de façon directe et simple, c'est-à-dire sans manipulation supplémentaire de l'échantillon, soit l'analyse immédiate de l'échantillon après un éventuel traitement, soit la conservation, de manière étanche, de l'échantillon éventuellement traité, en vue d'une analyse ultérieure.

La présente invention concerne un système d'analyse d'un échantillon comprenant un tel dispositif et un système de cassette à bandelettes adaptable au dispositif permettant la détection/quantification de l'analyte ou des analytes d'intérêt présent(s) dans l'échantillon sans pipetage supplémentaire. Ce système d'analyse permet ainsi à l'opérateur d'accomplir, avec le même matériel, toutes les opérations requises, et dans l'ordre nécessaire, pour obtenir ou afficher le résultat de la détermination de la présence de l'analyte ou des analytes d'intérêt, à partir du milieu susceptible de le ou de les contenir.

### ÉTAT DE LA TECHNIQUE ANTÉRIEURE

De nombreux travaux et études dans des domaines englobant notamment la recherche académique ; la recherche et les analyses, cliniques et diagnostiques, avec des essais réalisés sur des prélèvements de sang ou des biopsies cellulaires ou tissulaires; la surveillance environnementale; la surveillance civile; la sécurité alimentaire et notamment le contrôle qualité ; les analyses criminelles sur traces... impliquent le prélèvement d'échantillons en vue de leur analyse.

L'état de la technique connaît plusieurs brevets et demandes de brevet proposant déjà des dispositifs adaptés au prélèvement et au stockage d'échantillons. A titre d'exemples particuliers, on peut citer la demande internationale WO 2012/118392 [1] et la demande internationale WO 2008/030817 **[2].**

Plus particulièrement, le dispositif objet de la demande internationale WO 2012/118392 **[1]** est constitué d'un réceptacle avec un bouchon ayant une zone perçable et pouvant présenter une tige avec une matrice solide du type éponge ou tampon. Le réceptacle a un fond arrondi ou conique avec un ou plusieurs trou(s). Le fond du réceptacle est relié à un collecteur via un pas de vis ou des protubérances. Dans cette demande, l'échantillon est prélevé à l'aide de la tige fixée sur le bouchon ; puis, le bouchon est vissé sur le réceptacle et une solution pour diluer l'échantillon est introduite par la zone perçable. Après agitation, la solution contenant l'échantillon est transférée dans le collecteur par centrifugation. En d'autres termes, l'utilisation d'une centrifugeuse est obligatoire pour récupérer l'échantillon, rendant le dispositif incompatible avec une utilisation sur le terrain. De plus, le transfert de l'échantillon pour une analyse ultérieure nécessite une manipulation additionnelle telle qu'un pipetage.

Le dispositif objet de la demande internationale WO 2008/030817 **[2]** présente une enceinte dans laquelle se trouve une matrice solide du type éponge ou tampon liée à un piston. Une fois que cette matrice s'est imprégnée par capillarité de l'échantillon, l'enceinte est connectée à un collecteur et l'échantillon passe dans le collecteur par compression manuelle de la matrice. Le collecteur peut être déconnecté de l'enceinte avant d'être obturé par un bouchon et ce, afin de stocker l'échantillon en vue d'une analyse ultérieure. Cette déconnexion n'est toutefois pas sans risque de contamination ou de perte d'échantillon.

Les analyses ultérieures réalisées sur les échantillons prélevés et éventuellement stockés visent à détecter la présence ou l'absence d'un analyte particulier dans ces derniers. Ces analyses peuvent mettre en œuvre des capteurs immunologiques se présentant sous forme de bandelettes, utilisés couramment pour la détection de nombreux paramètres biologiques mais également pour permettre la détection d'agents pathogènes.

Un tel capteur également appelé test-bandelette est un système de détection simple avec très peu de manipulations à réaliser ; rapide, le résultat étant obtenu en moins de 30 min ; peu coûteux et qui peut être utilisé sur le terrain par des non spécialistes. Les bandelettes sont formées de 3 zones fixées ensemble sur un support plastique, avec (i) une zone d'absorption favorisant la migration, (ii) une zone de réaction, formée généralement d'une membrane de nitrocellulose et (iii) une zone de dépôt recevant l'échantillon.

On associe, souvent, à cette dernière, une dernière zone sur laquelle a été déposé et séché l'anticorps « traceur ». Cet anticorps dirigé spécifiquement contre l'analyte d'intérêt est lié à des molécules permettant d'obtenir un signal, en général colorimétrique ou fluorescent.

Sur la membrane de nitrocellulose, sont déposées deux lignes d'anticorps. La première de ces lignes dite ligne « test » est réalisée par dépôt d'anticorps spécifiques de l'analyte d'intérêt (anticorps de capture). C'est le signal obtenu au niveau de cette ligne qui indiquera la présence ou non de l'analyte d'intérêt à détecter. Dans certains cas, ce signal permettra également la quantification. La deuxième ligne dite ligne « contrôle » est constituée généralement par des anticorps dirigés contre l'anticorps traceur.

Lors de la réalisation du test, si l'analyte d'intérêt est présent dans l'échantillon, celui-ci va être reconnu par l'anticorps traceur présent au niveau de la zone de dépôt. Le complexe ainsi formé, va être capturé par le deuxième anticorps spécifique de l'analyte d'intérêt au niveau de la ligne test au cours de la migration. L'excès de l'anticorps traceur n'ayant pas réagi avec l'analyte d'intérêt sera lui capturé au niveau de la ligne contrôle par l'anticorps anti-anticorps traceur. Si l'analyte d'intérêt est absent de l'échantillon, aucun complexe anticorps traceur/analyte d'intérêt ne sera pas formé et, par conséquent, l'anticorps traceur ne sera pas immobilisé au niveau de la ligne test. Dans ce format, le test est donc positif si un signal apparaît au niveau des deux lignes et est négatif si un signal apparaît seulement au niveau de la ligne contrôle. Si, à la fin du test, aucun signal n'apparaît, même au niveau de la ligne contrôle, cela signifie que le test n'a pas été effectué dans les bonnes conditions ou que l'échantillon n'est pas analysable en l'état.

L'état de la technique connaît également des dispositifs associant prélèvement et analyse comme, par exemple, via des tests-bandelettes.

Par exemple, la demande internationale WO 95/08761 **[3]** propose un dispositif composé d'une structure creuse à l'intérieur de laquelle est placée une mèche/tampon absorbant. L'une des extrémités de cette structure permet la sortie de la mèche. A l'extrémité opposée, se situe le système d'analyse de l'échantillon. Pour prélever l'échantillon, la mèche est sortie de cette structure et absorbe l'échantillon liquide. Pour l'analyse, la mèche est mise en contact avec le système d'analyse. A noter que le dispositif décrit dans **[3]** ne possède pas de réservoir qui permette le stockage et le transport de l'échantillon ou encore une analyse de ce dernier différée dans le temps.

La demande internationale WO 2009/036168 **[4]** décrit un dispositif pour le prélèvement, l'analyse et le stockage d'un échantillon. Ce dispositif possède, d'une part, un tampon/éponge qui permet de prélever des échantillons liquides, surfaciques et d'air et, d'autre part, un réservoir contenant une solution tampon qui permet de laver le tampon/éponge et ainsi récupérer l'échantillon après avoir refermé le logement du tampon/éponge. Le déclenchement du lavage est réalisé par le manipulateur. L'échantillon est ensuite analysé par un test (exemple bandelette) contenu dans le dispositif. Même si le dispositif décrit dans **[4]** semble être simple d'utilisation, sa fabrication nécessite l'assemblage de nombreuses pièces dont certaines doivent être mobiles. La production d'un tel dispositif est donc compliquée et coûteuse.

La demande internationale WO 2009/153559 **[5]** décrit un dispositif constitué d'une partie femelle dans laquelle est inséré un absorbant et une partie mâle qui peut s'insérer dans la partie femelle. Au niveau de l'extrémité de la partie femelle opposée à celle de l'insertion de la partie mâle, une communication est possible vers l'extérieur. Un système de détection est situé au niveau de cette extrémité et analyse l'échantillon provenant de la partie femelle. L'échantillon surfacique est prélevé grâce à l'absorbant humidifié puis l'échantillon est transféré vers le système de détection, soit par compression de l'absorbant à l'aide de la partie mâle, soit par aspiration par le côté opposé à la partie mâle. Là encore, le dispositif de **[5]** ne possède pas de réservoir qui permette le stockage et le transport de l'échantillon ou encore une analyse de ce dernier différée dans le temps.

Enfin le dispositif objet de la demande de brevet US 2016/121322 **[6]** comprend un corps pourvu (i) d'une éponge apte à absorber un échantillon fluidique oral, (ii) d'une bandelette apte à recevoir ledit échantillon et (iii) d'une portion définissant un réservoir fluidique et un canal dans lequel se trouve la bandelette. Ce dispositif présente en outre une capsule apte à recouvrir partiellement le corps au moyen de pression/rotation et comprenant un réservoir fluidique contenant un fluide destiné à se mélanger à l'échantillon. Toutefois un stockage prolongé de l'échantillon dans un tel dispositif n'apparaît pas envisageable. US2010/089181A1 fait partie de l'état de la technique.

Les inventeurs se sont fixé pour but de proposer un dispositif simple d'utilisation, peu coûteux quant à sa réalisation et permettant, d'une part, le prélèvement facilité d'échantillons que ces derniers soient liquides ou surfaciques et, d'autre part, le transport et le stockage des échantillons en prévision d'une future analyse.

Les inventeurs se sont également fixé pour but de proposer un dispositif facilement adaptable sur des systèmes d'analyse comme des tests-bandelettes, afin de disposer d'un outil permettant d'effectuer facilement (sans nécessiter de nombreuses manipulations de l'opérateur) toutes les étapes du prélèvement au rendu de résultats.

Les inventeurs se sont également fixé pour but de proposer un dispositif dont les caractéristiques permettent de tester plusieurs analytes d'intérêt en même temps et de garantir des performances des tests améliorés par rapport aux résultats obtenus sur des systèmes d'analyse comme des tests-bandelettes classiques.

### EXPOSÉ DE L'INVENTION

La présente invention permet d'atteindre les buts que se sont fixés les inventeurs et de résoudre tout ou partie des problèmes techniques des dispositifs de prélèvement de l'art antérieur.

En effet, les présents inventeurs ont mis au point un dispositif simple d'utilisation et ne nécessitant pas un savoir-faire particulier à cet effet. Ce dispositif présente trois modules, connectables les uns aux autres, avec un bouchon/piston, un module de prélèvement et un réservoir, ledit dispositif facilitant le prélèvement d'un échantillon liquide ou surfacique. Ce dispositif permet le stockage de l'échantillon dans un volume hermétiquement clos impliquant le bouchon/piston, le module de prélèvement et le réservoir. De plus, sont autorisés et facilités le traitement de l'échantillon et notamment son incubation avec une molécule pouvant se fixer sur l'analyte et produisant un signal mesurable. Les différentes fonctions de ce dispositif facilitent la manipulation de l'échantillon et éventuellement permettent l'augmentation des signaux des tests de détection.

En particulier, la matrice poreuse, éventuellement compressible, présente dans le dispositif selon l'invention permet le recueil d'un échantillon liquide ou d'un échantillon sur une surface solide. La possibilité d'une incubation entre l'analyte présent dans l'échantillon et une molécule marquée se fixant sur l'analyte dans le dispositif de prélèvement ou le réservoir favorise l'interaction entre ces deux éléments et la capacité de stockage permet de réaliser l'analyse de l'échantillon au moment et à l'endroit voulus.

Enfin ce dispositif simplifie le dépôt de l'échantillon sur un système de détection/quantification tel que les tests-bandelettes, une connexion hermétique entre ce dispositif et la cassette contenant une ou des bandelette(s) réactive(s) permet d'assurer un dépôt simplifié et sécurisé.

Ainsi, la présente invention concerne un dispositif de prélèvement et éventuellement de traitement d'un échantillon, ledit dispositif comprenant :
une enceinte avec une première extrémité ouverte et une seconde extrémité présentant un orifice,
une matrice poreuse, disposée dans ladite enceinte et apte à récupérer ledit échantillon,
un bouchon connectable sur ladite première extrémité de ladite enceinte et présentant un piston assurant une fermeture étanche de ladite première extrémité de ladite enceinte, ledit bouchon étant déplaçable par rapport à ladite enceinte d'une position initiale à une position finale moyennant quoi ledit piston compresse ladite matrice poreuse ou ledit échantillon,
un réservoir connectable sur ladite seconde extrémité de ladite enceinte et en connexion fluidique avec ladite enceinte via ledit orifice de façon à récupérer l'échantillon restitué par ladite matrice poreuse sous l'effet de la compression de ladite matrice poreuse ou dudit échantillon par ledit piston, ledit réservoir présentant un ou plusieurs conduit(s) reliant le volume interne du réservoir à l'extérieur,
des moyens d'étanchéité étant présents entre ledit bouchon et ledit réservoir et
lorsque ledit bouchon et ledit réservoir sont connectés à ladite enceinte et que ledit bouchon est dans ladite position finale, ledit bouchon étant en contact direct avec ledit réservoir et le fermant de manière étanche et ledit ou lesdits conduit(s) étant obturé(s).

Le dispositif selon la présente invention comprend donc trois modules interagissant les uns avec les autres aux moyens de connexions mécaniques et/ou fluidiques.

Le premier de ces modules est le module central participant au recueil et/ou au prélèvement de l'échantillon : il comprend une enceinte et une matrice poreuse, compressible ou non, placée dans cette enceinte. Les expressions « premier module », « module préleveur » et « préleveur » sont équivalentes et utilisables de façon interchangeable dans la présente invention.

Typiquement, cette enceinte se présente sous forme cylindrique tubulaire à section circulaire avec une première extrémité ouverte et une seconde extrémité présentant un orifice, lesdites extrémités étant positionnées en regard l'une de l'autre.

La seconde extrémité de cette enceinte peut ne présenter qu'un seul orifice traversant ou, au contraire, présenter plusieurs orifices et notamment deux, trois, quatre ou une pluralité d'orifices traversants répartis, de façon régulière ou non, au niveau de la seconde extrémité. Dans un mode de réalisation particulier, l'orifice peut présenter un diamètre interne identique ou sensiblement identique au diamètre interne de la seconde extrémité de l'enceinte. Un tel mode de réalisation est notamment mis en œuvre lorsque la matrice poreuse est une membrane de filtration.

Dans le cadre de la présente invention, la matrice poreuse, compressible ou non, mise en œuvre peut être tout support solide apte, d'une part, à absorber et/ou à filtrer un échantillon et, d'autre part, à restituer cet échantillon en réponse à une compression de ladite matrice lorsqu'elle est poreuse ou de l'échantillon liquide lorsque la matrice poreuse est non compressible. Cette matrice est un support solide présentant des pores de taille régulière ou irrégulière, distribuées de façon aléatoire. Cette matrice se présente typiquement sous forme d'une éponge, d'une mousse, d'un tampon, lorsqu'elle est compressible ou sous forme d'une membrane de filtration, lorsqu'elle est non compressible. Cette matrice est avantageusement en un matériau comprenant cellulose, esters de cellulose, polyfluorure de vinyldène (PVDF), nitrocellulose, polycarbonate, élastomère ou un de leurs mélanges. L'homme du métier saura déterminer, sans effort inventif, le matériau le plus adapté pour la matrice poreuse et sa nature compressible ou non (mousse ou filtre) en fonction de la nature et notamment du caractère hydrophile ou hydrophobe de l'échantillon à prélever.

Typiquement la matrice poreuse présente une forme cylindrique pleine à section circulaire. Avantageusement, le diamètre de la matrice poreuse est identique ou sensiblement identique au diamètre intérieur de l'enceinte de forme cylindrique tubulaire de façon à ce que l'échantillon ne passe pas à côté de ladite matrice pour rejoindre le réservoir. En d'autres termes, il existe un contact direct entre la paroi latérale interne de l'enceinte et la matrice poreuse.

Lorsque la matrice est poreuse et compressible, avant que le bouchon ne soit connecté à l'extrémité ouverte de l'enceinte, la matrice peut dépasser i.e. émerger de l'extrémité ouverte de l'enceinte et ce, afin de faciliter le prélèvement direct de l'échantillon.

Dans un premier mode de réalisation, l'échantillon à prélever est un échantillon liquide. On entend par « échantillon liquide » toute solution naturelle ou synthétique, hydrophile ou hydrophobe, que l'on souhaite prélever aux moyens du dispositif de l'invention.

Ainsi, cet échantillon liquide peut être un fluide biologique ; un fluide végétal tel que sève, nectar et exsudat racinaire ; un prélèvement dans un milieu de culture ou dans un réacteur de culture biologique comme une culture cellulaire d'eucaryotes supérieurs, de levures, de champignons ou d'algues ; un liquide obtenu à partir d'une (ou plusieurs) cellule(s) animale(s) ou végétale(s) ; un liquide obtenu à partir d'un tissu animal ou végétal ; un prélèvement dans une matrice alimentaire ; un prélèvement dans un réacteur chimique ; de l'eau de ville, de rivière, d'étang, de lac, de mer, de tours aéro-réfrigérées; un prélèvement à partir d'un effluent industriel liquide; de l'eau usée provenant notamment d'élevages intensifs ou d'industries du domaine chimique, pharmaceutique, cosmétique ou nucléaire; un produit pharmaceutique; un produit cosmétique ; un parfum ; un échantillon de terre ou un de leurs mélanges.

Lorsque l'échantillon liquide est un fluide biologique, ce dernier est avantageusement choisi dans le groupe constitué par le sang tel que du sang entier ou du sang entier anti-coagulé, le sérum sanguin, le plasma sanguin, la lymphe, la salive, le crachat, les larmes, la sueur, le sperme, l'urine, les selles, le lait, le liquide céphalo-rachidien, le liquide interstitiel, un fluide isolé de moelle osseuse, un mucus ou fluide du tractus respiratoire, intestinal ou génito-urinaire, des extraits cellulaires, des extraits de tissus et des extraits d'organes. Ainsi, le fluide biologique peut être tout fluide naturellement sécrété ou excrété d'un corps humain ou animal ou tout fluide récupéré, à partir d'un corps humain ou animal, par toute technique connue de l'homme du métier telle qu'une extraction, un prélèvement ou un lavage. Les étapes de récupération et d'isolement de ces différents fluides à partir du corps humain ou animal sont réalisées préalablement à la mise en contact avec le dispositif selon la présente invention.

De même, si un des échantillons envisagés ne permet pas un prélèvement à l'aide du dispositif selon la présente invention, par exemple du fait de sa nature notamment solide, de sa concentration ou des éléments qu'il contient tels que résidus solides, déchets, suspension ou molécules interférentes, le prélèvement tel que défini ci-après comprend en outre une étape préalable de préparation de l'échantillon avec éventuellement une mise en solution de l'échantillon par les techniques connues de l'homme du métier telles que filtration, précipitation, dilution, distillation, mélange, concentration, lyse, etc. En variante, de telles étapes peuvent être mises en œuvre en utilisant le dispositif selon l'invention comme expliqué ci-après.

Lorsque l'échantillon à prélever est un échantillon liquide et que la matrice poreuse est compressible, le prélèvement peut consister à déposer, sur cette dernière, l'échantillon. Typiquement, l'échantillon est déposé sur la matrice poreuse et compressible par pipetage. En variante, la matrice peut être mise en contact avec l'échantillon liquide de façon à ce que ce dernier soit absorbé par capillarité par la matrice. Lorsque la matrice poreuse n'est pas compressible et se présente, par exemple, sous forme d'une membrane de filtration, l'échantillon est déposé dans l'enceinte contenant ou non une solution telle qu'un milieu de culture ou un tampon d'extraction. Cet échantillon liquide peut être issu de la mise en suspension d'un échantillon solide, comme, par exemple, un échantillon recueilli au moyen d'un écouvillon.

Dans un second mode de réalisation, l'échantillon à prélever est un échantillon surfacique. Par « échantillon surfacique », on entend un échantillon obtenu à partir d'un ensemble de composés organiques et/ou de composés inorganiques présents à la surface d'un support et se trouvant initialement sous forme sèche ou sensiblement sèche.

Lorsque l'échantillon à prélever est un échantillon surfacique, le prélèvement met en œuvre une matrice poreuse et compressible et consiste, tout d'abord, à imbiber cette dernière avec un tampon adapté et ce, soit par dépôt/pipetage, soit par absorption par capillarité puis à frotter la surface du support concerné avec la matrice poreuse ainsi imbibée.

Le deuxième module du dispositif selon l'invention correspond au bouchon/piston qui permet, lorsqu'il est connecté à l'enceinte du premier module, au niveau de l'extrémité ouverte de cette dernière, de compresser la matrice poreuse ou l'échantillon contenu dans l'enceinte moyennant quoi l'échantillon est extrait de la matrice poreuse et passe dans le volume interne du réservoir i.e. le troisième module. Les expressions « deuxième module », « module bouchon/piston » et « bouchon/piston » sont équivalentes et utilisables de façon interchangeable dans la présente invention.

Dans un premier mode de réalisation, l'échantillon passe de l'enceinte au réservoir grâce à la compression de la matrice poreuse et compressible par le piston, résultant du déplacement du bouchon par rapport à l'enceinte d'une position initiale à une position finale. Par « position initiale », on entend la position du bouchon lorsque ce dernier rentre en contact avec l'extrémité ouverte de l'enceinte. Par « position finale », on entend la position dans laquelle le déplacement du bouchon par rapport à l'enceinte est maximum. Lorsque le bouchon se déplace par rapport à l'enceinte, le piston coulisse à l'intérieur de l'enceinte. A noter que la matrice poreuse n'est pas fixée au piston.

Dans un second mode de réalisation, la matrice poreuse est non compressible, et c'est l'échantillon liquide ou mis en suspension et présent dans l'enceinte qui est comprimé ou poussé par le piston, lors du déplacement du bouchon par rapport à l'enceinte d'une position initiale à une position finale.

Typiquement, le piston se présente sous forme d'un cylindre fermé unique, à section circulaire, monté ou fixé sur le bouchon. Dans cette forme de mise en œuvre, le diamètre du piston est identique au diamètre intérieur de l'enceinte de forme cylindrique tubulaire du premier module. En variante, le piston peut présenter un diamètre inférieur au diamètre intérieur de l'enceinte. Dans ce cas, toutefois, la tête du piston en contact avec la matrice poreuse présente un joint d'étanchéité. Ce dernier peut s'étendre circonférentiellement autour de la tête du piston et/ou recouvrir cette tête. Quelle que soit la disposition de ce joint d'étanchéité, il présente un diamètre extérieur identique ou voire supérieur au diamètre intérieur de l'enceinte. Un joint d'étanchéité peut également être utilisé lorsque le diamètre du piston est identique au diamètre intérieur de l'enceinte. Dans toutes les variantes envisagées ci-dessus, le piston assure une fermeture étanche de la première extrémité de l'enceinte.

Tout moyen pour connecter, de façon amovible, le bouchon à la première extrémité de l'enceinte est utilisable dans le cadre de la présente invention. A titre d'exemples illustratifs, cette connexion peut impliquer un vissage, un clipsage, un encochage, un nervurage et/ou un montage à baïonnettes. Dans un mode de réalisation avantageux, cette connexion est réalisée par vissage et le bouchon est connectable sur la première extrémité de l'enceinte aux moyens de filetages. Dans ce mode de réalisation, le piston et le bouchon possèdent le même axe de rotation. Ainsi, le vissage du bouchon sur la première extrémité de l'enceinte entraîne la compression de la matrice poreuse ou de l'échantillon liquide contenu dans l'enceinte par ledit piston.

Le troisième module du dispositif selon l'invention correspond au réservoir qui, lorsqu'il est connecté à l'enceinte du premier module, au niveau de la seconde extrémité de cette dernière, récupère l'échantillon après passage de ce dernier à travers la matrice poreuse. Pour réaliser cette récupération, le réservoir est en connexion fluidique avec l'enceinte via l'orifice de la seconde extrémité de cette dernière.

Les expressions « troisième module », « module réservoir » et « réservoir » sont équivalentes et utilisables de façon interchangeable dans la présente invention.

Dans le cadre de la présente invention, le réservoir peut présenter n'importe quelle forme permettant une connexion hermétique à l'enceinte du premier module, au niveau de la seconde extrémité de cette dernière. Typiquement, ce réservoir se présente sous forme cylindrique avec une première extrémité ouverte. Cette première extrémité ouverte participe directement à la connexion à l'enceinte par vissage, clipsage, encochage, nervurage et/ou montage à baïonnette. Le diamètre extérieur du réservoir peut être inférieur, identique ou supérieur au diamètre extérieur de l'enceinte. Dans un mode de réalisation particulier, le diamètre extérieur du réservoir est supérieur au diamètre extérieur de l'enceinte.

Une des originalités du dispositif selon la présente invention réside dans le fait que, lorsque le bouchon est connecté à l'enceinte et se trouve dans la position finale telle que précédemment définie et lorsque le réservoir est connecté à l'enceinte, le bouchon ferme ou clôt ou engage, de manière étanche, le réservoir. Lorsque le bouchon est en contact direct avec le réservoir, des moyens d'étanchéité existent entre le bouchon et le réservoir. Dans cette organisation, le bouchon et le réservoir ferment, de façon étanche, le dispositif moyennant quoi ce dernier et l'échantillon qu'il contient peuvent être conservés ou stockés, de façon sécurisée. En particulier, dans ce mode de réalisation à savoir lorsque le bouchon est connecté à l'enceinte et se trouve dans la position finale telle que précédemment définie et lorsque le réservoir est connecté à l'enceinte, le bouchon et le réservoir forment un seul volume fermé étanche qui contient l'enceinte i.e. le premier module. Il est possible de renforcer encore l'étanchéité du volume ainsi formé et éviter une ouverture intempestive du dispositif en disposant au niveau de la jointure entre le bouchon et le réservoir un ruban adhésif et notamment un ruban adhésif friable.

Tous moyens d'étanchéité connus de l'homme du métier peuvent être mis en œuvre entre le bouchon et le réservoir. Dans un mode de réalisation particulier, ces moyens d'étanchéité impliquent un joint d'étanchéité, ce dernier pouvant être porté soit par le réservoir, soit par le bouchon. Dans un mode de réalisation particulier, ces moyens d'étanchéité se présentent sous forme d'un joint d'étanchéité porté par le bouchon. Dans ce cas de figure, le réservoir présente une cavité ou gorge dans laquelle le joint d'étanchéité peut se loger de manière étanche. En variante, le réservoir présente un logement annulaire auquel le joint d'étanchéité peut se caler de manière étanche. Typiquement, le joint d'étanchéité mis en œuvre dans le cadre de la présente invention est un joint d'étanchéité torique ou un joint d'étanchéité à lèvres.

Tout moyen pour connecter, de façon amovible ou inamovible, le réservoir à la seconde extrémité de l'enceinte est utilisable dans le cadre de la présente invention. En effet, il est envisageable, au cours du prélèvement et du traitement de l'échantillon, de remplacer un premier réservoir par un second réservoir et ce, comme illustré dans l'exemple IV.2.a ci-après. A titre d'exemples illustratifs, cette connexion peut impliquer un collage, un emboutissage, un vissage, un clipsage, un encochage, un nervurage et/ou un montage à baïonnettes. Ainsi, cette connexion met en œuvre une ou plusieurs encoche(s), une ou plusieurs saillie(s), un ou plusieurs ergot(s), une ou plusieurs protubérance(s), une ou plusieurs languette(s) et un ou plusieurs évidemment(s) au niveau de la seconde extrémité de l'enceinte et de la première extrémité du réservoir. Dans une variante de connexion inamovible du réservoir à la seconde extrémité de l'enceinte, le réservoir et l'enceinte peuvent former une structure unique.

Le réservoir du dispositif selon l'invention présente également un ou plusieurs conduit(s) reliant le volume interne du réservoir à l'extérieur. De cette façon, tout risque de surpression dans le volume interne du réservoir est évité lors de la compression de la matrice poreuse ou de l'échantillon par déplacement du bouchon par rapport à l'enceinte d'une position initiale à une position finale telles que précédemment définies.

Lorsque le bouchon et le réservoir sont connectés à l'enceinte et lorsque le bouchon se trouve dans la position finale, ce ou ces conduit(s) se trouve(nt) obturé(s), garantissant ainsi l'étanchéité du dispositif selon la présente invention.

Avantageusement, dans le dispositif selon l'invention, le réservoir présente en outre un orifice obturable. A l'état non obturé, cet orifice permet que tout ou partie de l'échantillon soit extrait du réservoir en vue d'une élimination ou d'une analyse. Au contraire, lorsque cet orifice est obturé, l'échantillon est maintenu dans le réservoir, ce qui est notamment le cas lors du stockage ou du transport de l'échantillon dans le dispositif selon la présente invention.

Cet orifice obturable se trouve au niveau de l'extrémité en regard de la première extrémité ouverte du réservoir. Cette seconde extrémité du réservoir peut ne présenter qu'un seul orifice obturable ou, au contraire, présenter plusieurs orifices et notamment deux, trois, quatre ou une pluralité d'orifices obturables répartis, de façon régulière ou non, au niveau de la seconde extrémité.

Le réservoir du dispositif selon l'invention présente donc des moyens pour obturer le ou les orifice(s) et ce, de façon amovible ou sécable. Ces moyens peuvent se trouver au niveau de la paroi interne ou externe du réservoir ou encore peuvent traverser le ou les orifice(s) obturable(s). Typiquement, des moyens pour obturer un orifice obturable se présentent sous forme d'un obturateur et en particulier d'un obturateur couplé à un picot disposé de façon amovible dans cet orifice.

Avantageusement, le dispositif selon l'invention présente en outre un orifice obturable par un obturateur amovible. Dans un mode de réalisation particulier, l'orifice obturable par un obturateur amovible, présent au niveau du réservoir est protégé par un bouchon moyennant quoi aucune ouverture intempestive de cet orifice n'interviendra. En effet, l'utilisateur devra procéder au dévissage du bouchon protecteur avant que l'orifice obturé ne puisse être ouvert.

Afin de traiter l'échantillon durant le prélèvement ou le stockage de ce dernier, le dispositif de l'invention peut comprendre en outre au moins un élément apte à traiter physiquement ou chimiquement cet échantillon.

Dans un mode de réalisation particulier, cet élément est une membrane de filtration. Cette dernière permet de séparer, d'isoler ou encore d'extraire des éléments comme des cellules présents dans l'échantillon du reste de l'échantillon. Cette membrane de filtration peut être placée dans l'enceinte entre la matrice poreuse compressible et la seconde extrémité de cette enceinte.

Dans un autre mode de réalisation, cet élément est un réactif chimique. A titre d'exemples de tels réactifs, on peut citer un tampon de dilution, un tampon de lyse, un tampon de lavage ou une molécule apte à réagir avec un élément susceptible d'être présent dans l'échantillon. Par « molécule apte à réagir avec un analyte d'intérêt », on entend aussi bien une molécule notamment du type enzyme apte à transformer cet élément en un autre composé ou à obtenir à partir de cet élément un autre composé qu'une molécule notamment du type anticorps apte à former avec un analyte d'intérêt un complexe ou paire de liaison. Ce réactif chimique peut être présent, dans le dispositif selon l'invention, préalablement au prélèvement, notamment sous forme séchée dans le réservoir, dans la matrice poreuse et/ou sur la matrice poreuse.

En variante, ce réactif chimique peut être apporté dans le dispositif après le prélèvement de l'échantillon. Dans ce cas, une fois l'échantillon dans le réservoir, on peut envisager de déconnecter le bouchon/piston de l'enceinte et de déposer ce réactif sur la matrice poreuse avant de reconnecter le bouchon à l'enceinte et de le ramener dans la position finale moyennant quoi le réactif chimique traverse la matrice poreuse et se retrouve dans le réservoir en présence de l'échantillon.

Dans une autre variante mettant en œuvre à la fois une matrice poreuse sous forme de membrane de filtration et un réactif chimique, on peut envisager que l'échantillon soit initialement traité par la membrane de filtration de façon à retenir des éléments d'intérêt au niveau de la membrane de filtration. Ce traitement consiste en la filtration de l'échantillon ; cette dernière peut être réalisée via la compression de l'échantillon en ramenant le bouchon/piston dans la position finale et/ou via une aspiration de l'échantillon. Dans ce dernier cas de figure, une dépression peut être réalisée au niveau de la seconde extrémité de l'enceinte, notamment en connectant l'orifice présent au niveau de cette extrémité à une pompe à vide ou à une seringue. Suite à cette filtration, les éléments de l'échantillon qui n'ont pas été retenus par la membrane à filtration peuvent être éliminés notamment en remplaçant le réservoir les contenant par un autre réservoir. Ensuite, le bouchon est déconnecté de l'enceinte. Le réactif chimique est alors déposé sur la matrice poreuse avant de reconnecter le bouchon à l'enceinte et de le ramener dans la position finale moyennant quoi le réactif chimique se retrouve en contact avec les éléments d'intérêt retenus au niveau de la membrane de filtration puis les produits de la réaction entre ces éléments et le réactif chimique passent dans le réservoir comme illustré à l'exemple IV.2.a ci-après.

Le fait que l'échantillon puisse être traité directement dans le dispositif selon l'invention présente de nombreux avantages. Tout d'abord, ce traitement ne nécessite aucune manipulation par un tiers de l'échantillon suite à son prélèvement ; la reproductibilité et la fiabilité des analyses s'en trouvent accrues. De plus, le traitement ayant lieu dans le dispositif, les temps de contact entre l'échantillon et les réactifs chimiques sont plus longs que les temps de contact sur les tests-bandelettes classiques, ce qui participe à l'augmentation du signal détecté.

A noter que les matériaux utilisés pour la fabrication du dispositif de prélèvement et éventuellement de traitement selon l'invention et notamment utilisés pour les parties susceptibles d'être en contact avec l'échantillon interagissent peu et typiquement sont choisis pour ne pas interagir avec le ou les analyte(s) d'intérêt à étudier afin de minimiser les problèmes de sorption et de désorption sur les surfaces d'échange que fournit le dispositif.

La présente invention concerne également un système pour détecter et éventuellement quantifier un analyte d'intérêt susceptible d'être présent dans un échantillon, le système comprenant :
- une cassette présentant une ouverture,
- au moins une bandelette apte à recevoir ledit échantillon via ladite ouverture et présentant un indicateur visuel indiquant la présence d'un analyte d'intérêt dans ledit échantillon étant placée dans ladite cassette et
- un dispositif tel que précédemment défini et notamment dont le réservoir présente un orifice obturable, ledit dispositif étant en connexion fluidique avec la cassette par l'intermédiaire de l'orifice obturable passant d'une configuration fermée à une configuration ouverte en réponse à la connexion de l'ouverture de la cassette avec l'orifice obturable du dispositif.

Dans un tel système, le dépôt de l'échantillon au niveau de la cassette se trouve facilité.

L'analyte à détecter et éventuellement à quantifier est présent dans l'échantillon liquide ou surfacique préalablement au prélèvement ou se trouve dans cet échantillon suite à son traitement dans le dispositif selon l'invention. L'analyte à détecter peut être choisi dans le groupe constitué par une molécule d'intérêt biologique ; une molécule d'intérêt pharmacologique ; une toxine ; un peptide ; une protéine ; une glycoprotéine ; une enzyme ; un substrat enzymatique ; un récepteur nucléaire ou membranaire ; un agoniste ou un antagoniste d'un récepteur nucléaire ou membranaire ; une hormone ; un anticorps polyclonal ou monoclonal ; un fragment d'anticorps tel qu'un fragment Fab, F(ab')₂, Fv ou un domaine hypervariable ou CDR pour « Complementarity Determining Région » ; une bactérie et un virus. En fonction de l'analyte à détecter, l'homme du métier saura déterminer, sans effort inventif, les composés marqués et les composés non marqués à utiliser pour obtenir une indication visuelle au niveau de la cassette lorsque cet analyte est présent dans l'échantillon étudié.

Une cassette dans laquelle est placée au moins une bandelette apte à recevoir un échantillon et présentant un indicateur visuel indiquant la présence d'un analyte d'intérêt dans cet échantillon est connue en soi dans le domaine de la détection et de l'éventuelle quantification d'un analyte d'intérêt. L'originalité de cette cassette dans le cadre de la présente invention réside dans le fait qu'elle présente une ouverture adaptée pour se connecter à l'orifice obturable du réservoir du dispositif contenant l'échantillon et permettre l'ouverture de cet orifice et de fait l'introduction de l'échantillon dans la cassette. Ainsi, lorsque l'orifice obturable est obturé par un obturateur sécable présentant un picot, la connexion du dispositif sur la cassette entraîne la compression du picot relié à l'obturateur sécable ce qui provoque l'ouverture de l'orifice. De fait, l'ouverture de l'orifice est contrôlée et ne peut se produire que lorsque le dispositif est connecté à la cassette. Cette particularité sécurise le dépôt de l'échantillon en évitant tout risque de débordement ou de dépôt en dehors de l'utilisation de la cassette disposant de l'ouverture adaptée à l'orifice obturable du réservoir.

Tout moyen pour connecter, de façon amovible, l'ouverture de la cassette à l'orifice obturable du réservoir du dispositif est utilisable dans le cadre de la présente invention. A titre d'exemples illustratifs, cette connexion peut impliquer un vissage, un clipsage, un encochage, un nervurage et/ou un montage à baïonnettes. Dans un mode de réalisation avantageux, cette connexion est réalisée par vissage et l'ouverture de la cassette est connectable sur l'orifice du réservoir du dispositif aux moyens de filetages.

Comme une grande quantité d'échantillons peut être introduite dans la cassette du fait du dispositif et du volume interne du réservoir utilisé pour prélever cet échantillon, la cassette mise en œuvre peut être configurée pour tester différents analytes d'intérêt et ce, sans perte de sensibilité. A cet effet, cette cassette comprend plusieurs bandelettes et notamment deux, trois, quatre, cinq ou plus bandelettes.

La présente invention concerne enfin un kit d'éléments comprenant les deux éléments formant le système d'analyse tel que précédemment décrit, sous une forme déconnectée l'un de l'autre et dont l'orifice du dispositif participant à cette connexion est protégé par un bouchon, l'ouverture de la cassette pouvant, quant à elle, être ou non protégée par un bouchon. En d'autres termes, la présente invention concerne un kit d'éléments comprenant :
- une cassette présentant une ouverture éventuellement protégée par un bouchon et au moins une bandelette apte à recevoir ledit échantillon via ladite ouverture et présentant un indicateur visuel indiquant la présence d'un analyte d'intérêt dans ledit échantillon étant placée dans ladite cassette et
- un dispositif tel que précédemment défini et notamment dont le réservoir présente un orifice obturable protégé par un bouchon.

D'autres caractéristiques et avantages de la présente invention apparaîtront encore à l'homme du métier à la lecture des exemples ci-dessous donnés à titre illustratif et non limitatif, en référence aux figures annexées.

### BRÈVE DESCRIPTION DES DESSINS

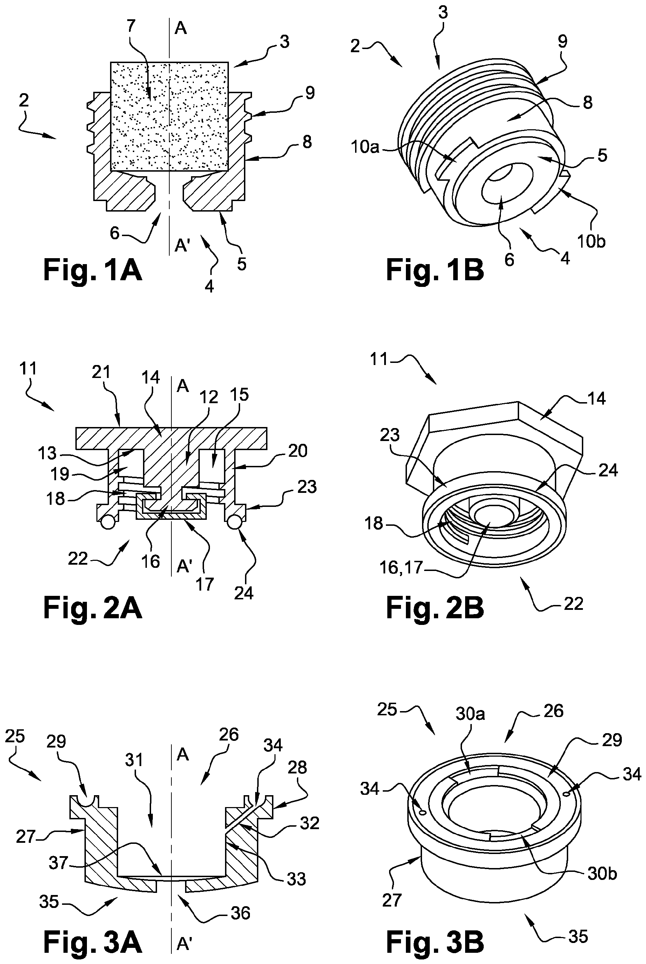

La Figure 1 concerne le premier module du dispositif de prélèvement et éventuellement de traitement selon la présente invention. La Figure 1A est une vue schématique partielle en coupe longitudinale du premier module du dispositif de prélèvement et éventuellement de traitement selon la présente invention. La Figure 1B est une vue schématique partielle en perspective du premier module du dispositif de prélèvement et éventuellement de traitement selon la présente invention.
La Figure 2 concerne le deuxième module du dispositif de prélèvement et éventuellement de traitement selon la présente invention. La Figure 2A est une vue schématique partielle en coupe longitudinale du deuxième module du dispositif de prélèvement et éventuellement de traitement selon la présente invention. La Figure 2B est une vue schématique partielle en perspective du deuxième module du dispositif de prélèvement et éventuellement de traitement selon la présente invention.
La Figure 3 concerne le troisième module du dispositif de prélèvement et éventuellement de traitement selon la présente invention. La Figure 3A est une vue schématique partielle en coupe longitudinale du troisième module du dispositif de prélèvement et éventuellement de traitement selon la présente invention. La Figure 3B est une vue schématique partielle en perspective du troisième module du dispositif de prélèvement et éventuellement de traitement selon la présente invention.

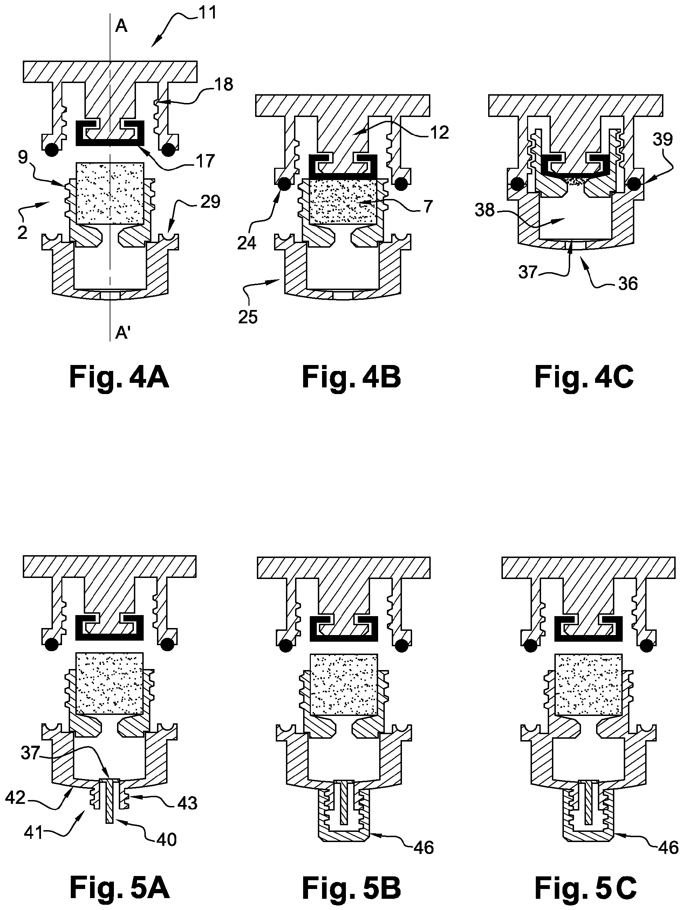

La Figure 4 propose des représentations schématiques partielles en coupe longitudinale du dispositif selon l'invention en cours d'utilisation. Les Figures 4A, 4B et 4C correspondent au dispositif avant le prélèvement ou juste après le prélèvement, au dispositif dans lequel le bouchon/piston est dans la position dite « position initiale » et au dispositif dans lequel le bouchon/piston est dans la position dite « position finale ».
La Figure 5 propose des représentations schématiques partielles en coupe longitudinale du dispositif selon l'invention dans lequel l'obturateur non protégé (Figure 5A) ou protégé par un bouchon (Figure 5B) est adapté pour une utilisation de ce dispositif dans un système d'analyse selon l'invention. La Figure 5C est une variante de la Figure 5B dans laquelle le réservoir et l'enceinte forment une structure unique.

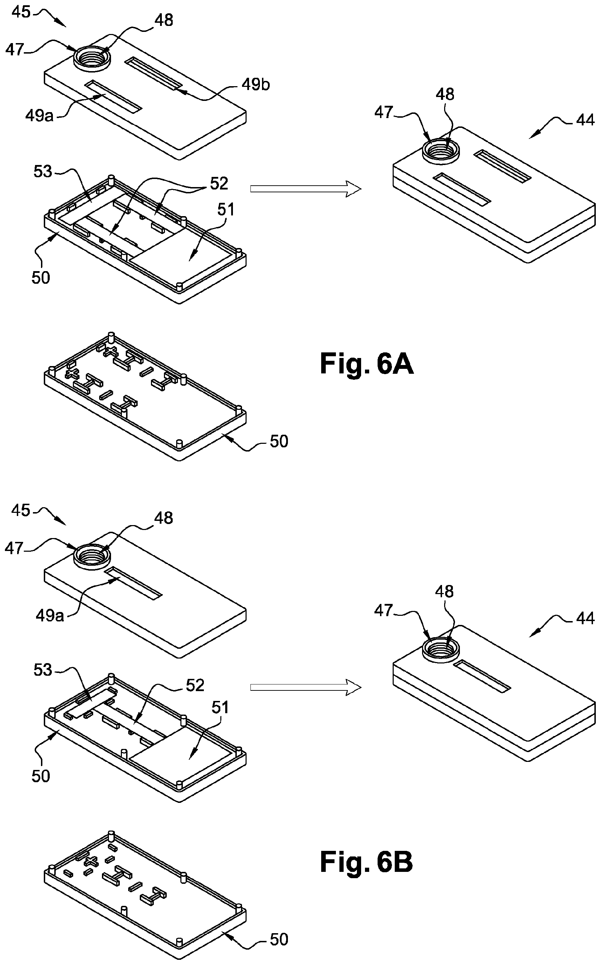

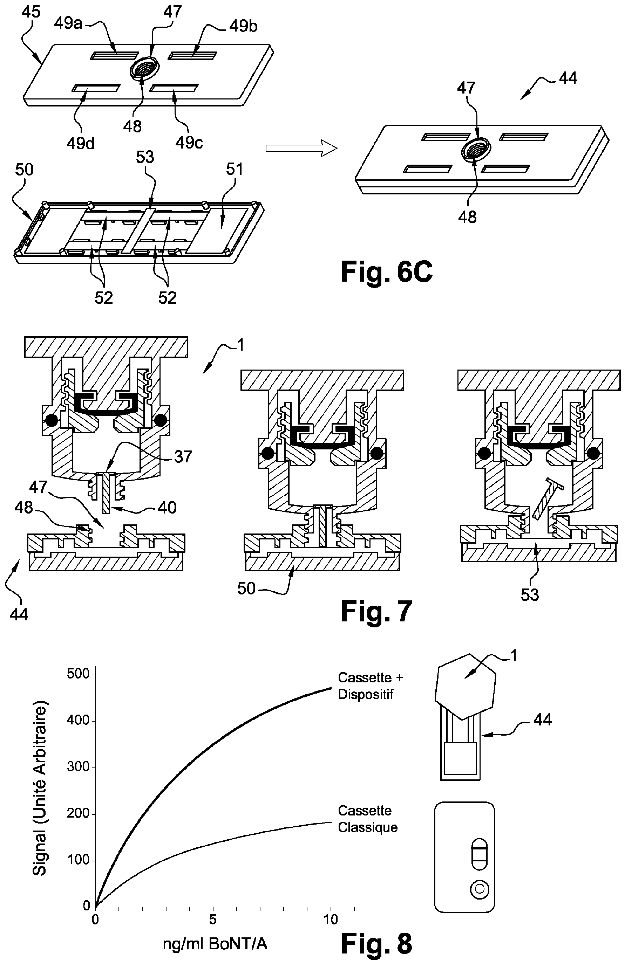

La Figure 6 présente différentes cassettes utilisables dans le système d'analyse selon l'invention, ces dernières comprenant deux bandelettes (Figure 6A), une seule bandelette (Figure 6B) ou quatre bandelettes (Figure 6C).
La Figure 7 présente une série séquentielle de représentations schématiques partielles en coupe longitudinale du dispositif selon l'invention lors de la connexion de ce dernier à une cassette telle que mise en œuvre dans le système d'analyse selon l'invention.
La Figure 8 est une comparaison du signal obtenu avec un système d'analyse selon l'invention ou avec une cassette de type test-bandelette classique pour différentes concentrations en toxine botulique.

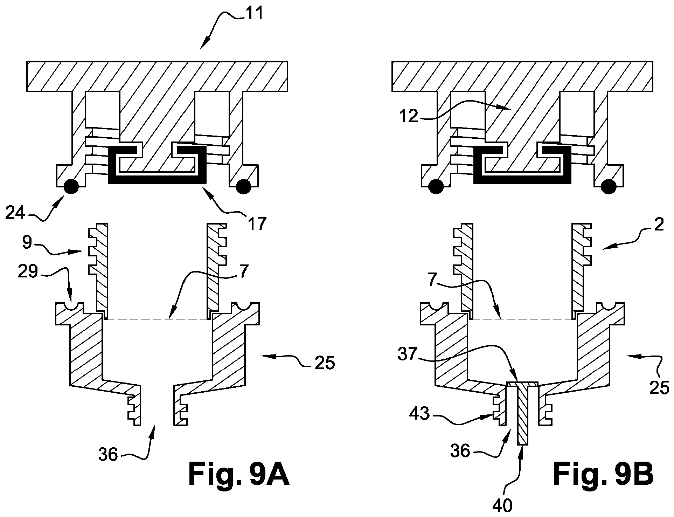

La Figure 9 propose des représentations schématiques partielles en coupe longitudinale de variantes du dispositif selon l'invention. La Figure 9A et 9B correspondent à un dispositif selon l'invention dans lequel la matrice poreuse se présente sous forme d'une membrane de filtration avec un réservoir dont l'orifice obturable est en position non obturée (Figure 9A) ou avec un réservoir dont l'orifice obturable est obturée par un obturateur avec picot (Figure 9B).

### EXPOSÉ DÉTAILLÉ DE MODES DE RÉALISATION PARTICULIERS

### I. Dispositif selon l'invention.

### I.1. Description.

Dans tout ce qui suit, le terme « radial » est défini par rapport à l'axe **AA'** du dispositif selon l'invention **1.**

Les Figures 1A et 1B représentent le premier module du dispositif de prélèvement et éventuellement de traitement selon l'invention. Ce premier module se présente sous forme d'une enceinte **2** correspondant à une pièce en plastique (résine photopolymérisable, impression 3D) de hauteur de 1,3 cm, cylindrique tubulaire à section circulaire et présentant une première extrémité ouverte **3** et une seconde extrémité **4,** lesdites extrémités étant positionnées en regard l'une de l'autre. Cette seconde extrémité est fermée par une paroi **5** percée d'un seul orifice **6.** A l'intérieur du cylindre formant l'enceinte, se trouve une matrice poreuse et compressible **7** du type éponge ou mousse. La matrice poreuse et compressible **7** présente une forme cylindrique pleine à section circulaire et son diamètre est identique ou sensiblement identique au diamètre intérieur de l'enceinte de forme cylindrique tubulaire. En absence d'un quelconque élément connecté à la première extrémité ouverte **3,** la matrice poreuse et compressible **7** dépasse légèrement de cette ouverture. Enfin, en vue de la connexion de ce premier module au deuxième module et au troisième module du dispositif selon l'invention, la surface extérieure **8** du cylindre formant l'enceinte présente, en partie haute i.e. du côté de la première extrémité **3,** un filetage ou pas de vis **9** permettant le vissage du deuxième module et, en partie basse i.e. du côté de la seconde extrémité **4,** des ergots (ou protubérances) **10a** et **10b** pour la connexion au troisième module.

Les Figures 2A et 2B représentent le deuxième module du dispositif de prélèvement et éventuellement de traitement selon l'invention. Ce deuxième module correspond à un bouchon/piston **11.** Le piston **12** de ce module se présente sous forme d'un cylindre fermé unique, à section circulaire, d'une hauteur de 1,7 cm monté i.e. fixé sur la face interne **13** de l'extrémité fermée **14** du bouchon et s'étendant dans la cavité du bouchon **15.** La tête du piston **16** présente une matière souple **17** de type caoutchouc faisant office de joint d'étanchéité, cette matière recouvrant la tête du piston et s'étendant circonférentiellement autour de cette dernière. Comme le bouchon/piston **11** se connecte à la première extrémité **3** de l'enceinte par vissage, un filetage ou pas de vis **18** est pratiqué à l'intérieur de la cavité **15** du bouchon, sur la surface intérieure **19** de la paroi latérale **20.** Ainsi le filetage **18** permet l'assemblage du premier module **2** avec le deuxième module **11** par vissage sur le filetage correspondant **9** pratiqué sur la surface extérieure **8** du cylindre formant l'enceinte. Dans ce mode de réalisation, le piston et le bouchon possèdent le même axe de rotation correspondant à l'axe AA'. La face extérieure **21** de l'extrémité fermée du bouchon **14** permet l'inscription d'informations concernant l'échantillon prélevé. Le bouchon présente, en regard de son extrémité fermée **14,** une extrémité ouverte **22.** Au niveau de cette extrémité ouverte **22,** la paroi latérale **20** présente un épaulement **23** en saillie radiale. Cet épaulement **23** porte un joint d'étanchéité **24** tel qu'un joint d'étanchéité torique. Les différents éléments formant ce deuxième module et notamment l'extrémité fermée **14,** la paroi latérale **20** et le piston **11** sont en plastique (résine photopolymérisable, impression 3D).

Les Figures 3A et 3B représentent le troisième module du dispositif de prélèvement et éventuellement de traitement selon l'invention. Ce troisième module se présente sous forme d'un réservoir **25** de forme cylindrique tubulaire à section circulaire, d'une hauteur de 1,3 cm, avec une première extrémité ouverte **26.** Cette première extrémité ouverte **26** participe directement à la connexion à l'enceinte **2.** Au niveau de la première extrémité ouverte **26,** le diamètre extérieur du réservoir **25** est supérieur au diamètre extérieur de l'enceinte **2.** La paroi latérale **27** du réservoir **25** présente, au niveau de cette extrémité ouverte **26,** un épaulement **28** en saillie radiale. Le diamètre extérieur de cet épaulement **28** est identique ou sensiblement identique au diamètre extérieur de l'épaulement **23** du bouchon/piston **11.** De plus, l'épaulement **28** présente une cavité hémisphérique ou gorge **29** dans laquelle le joint d'étanchéité **24** porté par l'épaulement **23** peut se loger de manière étanche. Cet épaulement **28** présente également des encoches **30a** et **30b** qui participent avec les ergots **10a** et **10b** à l'assemblage du troisième module avec le premier module par encochage ou emboutissage. L'intérieur ou volume interne **31** du réservoir est relié à l'extérieur par un ou plusieurs conduits **32.** Ce ou ces conduit(s) **32** est/sont raccordé(s) au niveau de la surface interne **33** de la paroi latérale **27** du réservoir **25** et débouche(nt) au niveau d'un ou de plusieurs orifice(s) **34** présent(s) au niveau de l'épaulement **28** et plus particulièrement au niveau de la cavité hémisphérique ou gorge **29.** Enfin, en regard de la première extrémité ouverte **26,** se trouve une paroi ou seconde extrémité **35** percée d'un orifice **36** obturé par un obturateur **37.**

### 1.2. Fonctionnement.

### A. Prélèvement d'un échantillon liquide.

L'échantillon liquide est déposé sur la matrice poreuse et compressible **7** qui absorbe ce dernier (Figure 4A). Après récupération de l'échantillon, le bouchon/piston **11** est vissé sur l'enceinte **2** via les filetages **9** et **18** moyennant quoi le bouchon/piston se trouve dans la position dite « position initiale » (Figure 4B). En s'enfonçant dans l'enceinte **2,** le piston **12** compresse le matrice poreuse et compressible **7** ce qui provoque le transfert de l'échantillon dans le réservoir **25.** La surpression dans le réservoir est évitée grâce aux conduits **32** qui permettent à l'air d'être expulsé à l'extérieur. Le vissage du bouchon/ piston **11** sur l'enceinte **2** est effectué jusqu'à l'insertion complète du joint d'étanchéité torique **24** dans la cavité hémisphérique ou gorge **29** moyennant quoi le bouchon/piston se trouve dans la position dite « position finale » (Figure 4C). L'insertion du joint **24** dans la cavité **29** ferme de façon hermétique les conduits **32.** A la fin de l'opération (Figure 4C), l'échantillon est contenu dans le réservoir **25** de façon hermétique et des éléments ont pu y être ajoutés ou retirés. Le réservoir **25** et le bouchon/piston **11** sont couplés l'un à l'autre de manière étanche créant un volume interne fermé étanche **38** dans lequel se trouve l'enceinte **2.** L'échantillon peut alors être stocké ou transporté de façon sécurisée. Un ruban adhésif friable non représenté peut être disposé au niveau de la jointure **39** entre le bouchon/piston **11** et le réservoir **25** pour garantir la non-ouverture du dispositif entre le prélèvement de l'échantillon et son analyse.

### B. Prélèvement d'un échantillon surfacique.

Une solution tampon est déposée sur la matrice poreuse et compressible **7** qui absorbe cette dernière. On frotte la surface, sur laquelle on suspecte la présence de la substance que l'on veut analyser, avec l'extrémité de la matrice poreuse et compressible **7** qui dépasse de l'enceinte **2.** Le bouchon/piston **11** est vissé sur l'enceinte **2** puis la suite des opérations est similaire à celle décrite pour un échantillon liquide.

### II. Système d'analyse selon la présente invention.

Pour l'utilisation d'un dispositif **1** selon l'invention dans un système d'analyse à cassette et tests-bandelettes, l'obturateur **37** du réservoir **25** est relié à un picot **40** qui est entouré par un petit cylindre **41** fixé sur la face externe **42** du fond du réservoir **25.** Sur la face externe de ce cylindre **41,** a été réalisé un pas de vis ou filetage **43** (Figure 5A) qui permet de visser le réservoir **25** sur la partie supérieure **45** d'une cassette pour bandelette **44.** Pour ne pas casser le picot **40** durant le prélèvement ou le stockage de l'échantillon, un bouchon **46** de protection est vissé sur le cylindre qui l'entoure (Figure 5B). La Figure 5C est une variante de la Figure 5B dans laquelle le réservoir est connecté de façon inamovible à la seconde extrémité de l'enceinte, le réservoir et l'enceinte formant une structure unique.

Après le prélèvement de l'échantillon et son transfert par vissage du bouchon/piston **11** dans le réservoir **25,** celui-ci pourra être déposé sur la/les bandelettes lorsque le manipulateur le désirera. Pour faciliter cette opération, le système d'analyse selon l'invention met en œuvre des cassettes particulières. En effet, elles possèdent une ouverture **47** pour le dépôt avec un pas de vis **48** qui correspond au pas de vis **43** du cylindre **41** entourant le picot **40** relié à l'obturateur **37** du réservoir **25.**

Les autres éléments de ces cassettes **44** sont les mêmes que ceux présents dans les cassettes de l'état de la technique (Figure 6). Par conséquent, on retrouve, dans les cassettes **44** de l'invention,
- une partie supérieure **45** formant capot et comprenant, outre l'ouverture **47** précédemment décrite, des fenêtres de lecture des résultats **49a, 49b, 49c** et **49d,** et
- une partie inférieure **50** où se trouvent la ou les bandelettes formées de 3 zones fixées ensemble sur un support plastique, avec (i) une zone d'absorption **51** favorisant la migration, (ii) une zone de réaction **52,** formée généralement d'une membrane de nitrocellulose et (iii) une zone de dépôt **53** recevant l'échantillon.

A titre d'exemples, les cassettes **44** mises en œuvre dans le système d'analyse selon l'invention peuvent permettre l'utilisation de deux bandelettes simultanément (Figure 6A), d'une seule bandelette (Figure 6B) ou de quatre bandelettes simultanément (Figure 6C) peuvent être également utilisées.

Pour le dépôt de l'échantillon sur le système d'analyse selon l'invention, le manipulateur doit dévisser le bouchon de protection **46** et visser le dispositif **1** sur la cassette **44.** Ce faisant, il comprime le picot **40** contre la partie basse **50** de la cassette **44** et ainsi détache l'obturateur **37** du fond du réservoir **25** ce qui déclenche le dépôt de l'échantillon sur la zone de dépôt **53** de la bandelette (Figure 7).

### III. Validation du dispositif et du système d'analyse selon l'invention.

L'ensemble des expériences de validation ont été réalisées avec des bandelettes pour la détection de la toxine botulique A.

### III.1. Taux de récupération d'un échantillon liquide ou surfacique avec le dispositif.

### A. Matériel et méthode.

Le tampon bandelette a pour composition : Tampon Tris/HCL 0,1 M pH 8 + 0,15 M NaCl + 0,5% Tween 20 + 1% Chaps + 0,01% azide de sodium.

Le tampon EIA a pour composition : Tampon phosphate de potassium 0,1 M pH 7.4 + BSA 0,1% + NaCl 0,15 M + 0.01% azide de sodium.

On prépare 4 ml de toxine botulique à 3 ng/ml dans du tampon EIA. Un ml de cette solution est déposé dans deux coupelles en plastique et laissé sécher 24 h sous une hotte.

Le contenu d'une coupelle est repris avec 1 ml de tampon bandelette. Cent µl de cette solution sont prélevés (échantillon A) et 900 µl sont déposés sur le matériau poreux du dispositif puis récupérés à la sortie du réservoir après utilisation du dispositif (échantillon B).

Un ml de tampon bandelette est déposé sur le matériau poreux du dispositif. On récupère le contenu de la 2^{ième} coupelle en frottant la surface de la coupelle avec le matériau poreux. Après utilisation du dispositif, on récupère la solution à la sortie du réservoir (échantillon C). Neuf cents µl de la solution de toxine botulique sont déposés directement sur le dispositif. Cette solution est récupérée à la sortie du réservoir après utilisation du dispositif (échantillon E). La solution de toxine botulique A (échantillon D) ainsi que les autres échantillons sont quantifiés par dosage immunologique.

### B. Résultats.

Les résultats du dosage immunologique en fonction de l'échantillon sont donnés dans le Tableau 1 ci-après.

**Tableau 1**

| Echantillons | Concentration |
|---|---|
| A | 2,4 ng/ml |
| B | 2,4 ng/ml |
| C | 1,8 ng/ml |
| D | 3,4 ng/ml |
| E | 3,4 ng/ml |

Le taux de récupération d'un échantillon liquide après utilisation du dispositif correspond à : (D/E)x100 et (B/A)x100. On obtient un taux de 100% pour les deux mesures. Le taux de récupération d'un échantillon surfacique correspond à : (C/E)x100, c'est-à-dire 53%. Cette valeur est proche de celle obtenue en re-solubilisant l'échantillon (A/E)x100 : 70%.

### C. Analyse.

Cette expérience montre que le dispositif permet de traiter un échantillon liquide sans aucune perte.

D'autre part, on montre que le dispositif permet de récupérer 53% d'un échantillon surfacique. Le taux obtenu en re-solubilisant l'échantillon montre qu'il est difficile de récupérer la totalité d'un échantillon séché sur une surface (70%). En effet, une part plus ou moins importante des molécules de cet échantillon, en fonction de la nature de l'échantillon et de la surface, est absorbée sur cette dernière et n'est plus disponible pour une analyse.

### III.2. Comparaison test-bandelette classique et système d'analyse selon l'invention.

### A. Protocole.

Dans cette étude, on utilise les mêmes bandelettes dans les deux formats. Cependant, pour les tests utilisant le dispositif selon l'invention, l'anticorps traceur est séché sur une membrane de fibre de verre qui est placée entre le matériau poreux et le fond du collecteur. Pour les tests classiques, cet anticorps traceur est séché sur une membrane en fibre de verre placée entre la zone de dépôt et la zone de détection (position utilisée dans les tests commerciaux). Pour chaque dispositif, on sèche 1 ml de tampon bandelette dans le matériau poreux.

Pour les tests classiques, on utilise des cassettes contenant une bandelette avec un orifice de dépôt sans pas de vis. Pour les tests utilisant le dispositif selon l'invention, on utilise des cassettes contenant 2 bandelettes (Figure 6A) et avec un orifice avec pas de vis, suivant le protocole décrit précédemment.

Pour les tests classiques, on dépose 100 µl d'échantillon. Pour les tests utilisant le dispositif selon l'invention, on dépose 1 ml d'échantillon sur le matériau poreux et l'échantillon est stocké 5 min dans le réservoir avant dépôt sur la cassette.

On dépose des solutions avec différentes concentrations en toxine botulique A (0 ; 0.3 ; 1; 3 et 10 ng/ml. Les signaux obtenus au niveau de la ligne test sont lus avec un lecteur de bandelette (Qiagen).

### B. Résultats.

Les signaux obtenus via un lecteur de bandelette (Qiagen) exprimés sous forme d'unités arbitraires sont présentés à la Figure 8.

### C. Analyse.

On observe sur la Figure 8 que l'utilisation du dispositif et du système d'analyse selon l'invention permet d'augmenter fortement les signaux obtenus au niveau de la ligne test pour une même concentration en comparaison avec les signaux obtenus avec un test classique.

### III.3. Influence de la distance entre zone de dépôt et ligne test.

Dans le cadre de détection multiplex (plusieurs cibles détectées simultanément) sur une même bandelette, il est nécessaire de réaliser plusieurs lignes tests. Ces différentes lignes ne seront donc pas à la même distance de la zone de dépôt.

Pour les tests classiques, l'anticorps traceur se situe après la zone de dépôt, le temps de contact entre l'anticorps traceur et l'échantillon avant d'atteindre la ligne test sera donc fonction de la distance de cette dernière par rapport à la zone de dépôt. Dans cette expérience, les distances indiquées correspondent à la distance entre le début de la zone de détection et la ligne test.

### A. Protocole.

Des bandelettes sont préparées avec des lignes tests à différentes distances du début de la zone de détection. Les traceurs sont préparés comme pour l'expérience précédente. Pour les tests classiques, on utilise des cassettes contenant une bandelette avec un orifice de dépôt sans pas de vis. Pour les tests avec dispositif selon l'invention, on utilise des cassettes contenant 2 bandelettes (Figure 6A) et avec un orifice avec pas de vis. Le test est effectué suivant le protocole décrit précédemment.

Pour les tests classiques, on dépose 100 µl d'échantillon et on réalise deux tests pour chaque distance. Pour les tests avec dispositif on dépose 1 ml d'échantillon sur le matériau poreux et l'échantillon est stocké 5 minutes dans le réservoir avant dépôt sur la cassette.

On dépose des solutions de toxine botulique à 3 ng/ml. Les signaux obtenus au niveau de la ligne test sont lus avec un lecteur de bandelette (Qiagen).

### B. Résultats.

Les signaux obtenus via un lecteur de bandelette (Qiagen) exprimés sous forme d'unités arbitraires sont présentés dans le Tableau 2 ci-dessous.

**Tableau 2**

| | Cassette classique | | Cassette avec dispositif selon l'invention | |
|---|---|---|---|---|
| Distance (d) | Bandelette 1 | Bandelette 2 | Bandelette 1 | Bandelette 2 |
| 5 mm | **39** | **58** | **304** | **338** |
| 13 mm | **120** | **102** | **355** | **297** |
| 17 mm | **144** | **112** | **332** | **328** |

### C. Analyse.

On observe dans le Tableau 2 que les signaux obtenus avec les tests classiques augmentent avec la distance entre la ligne test et la zone de dépôt. Contrairement aux tests effectués avec le dispositif selon l'invention pour lesquels les signaux restent identiques quelle que soit la distance de la ligne test. Ainsi, l'homogénéité des signaux quelle que soit la distance entre le dépôt et la ligne test permet la réalisation de test multiplex sans perte de signal et donc de sensibilité.

D'autre part on remarque pour les tests avec dispositif selon l'invention que les valeurs des signaux sont identiques sur les bandelettes 1 et 2 contenues dans la même cassette, cela démontre que l'échantillon se réparti de façon équitable sur les deux bandelettes au cours de la migration.

Cette expérience confirme également les résultats présentés au point II.2.C montrant que les signaux obtenus avec le dispositif sont plus élevés que ceux obtenus avec un test classique.

### D. Conclusion.

Le dispositif selon l'invention permet une collecte simplifiée d'un échantillon liquide ou surfacique avec de bon taux de récupération. Il permet le stockage et le transport sécurisé de celui-ci. D'autre part, son association avec des cassettes pour des bandelettes adaptées, permet d'augmenter les signaux obtenus et autorise le développement de test multiplex sans perte de sensibilité. Il permet également le dépôt de l'échantillon sur la/les bandelettes sans pipetage.

### IV. Exemple d'utilisation du dispositif et du système d'analyse selon l'invention pour la détection d'enzymes de résistance aux antibiotiques.

### IV.1. Modifications du dispositif selon l'invention.

Pour cet exemple, la matrice poreuse **7** est une membrane de filtration de 0,45 µm insérée au fond de l'enceinte **2** dont la seconde extrémité **4** présente un diamètre interne égal au diamètre de l'orifice **6.** La liaison entre l'enceinte **2** et le réservoir **25** se fait grâce à un système d'ergots **10a** et **10b** qui autorise leur séparation à volonté. Deux types de réservoirs **25** sont utilisés : un réservoir **25** dont l'orifice **36** est ouvert i.e. sans obturateur (Figure 9A) et un réservoir **25** dont l'orifice **36** est fermé par un obturateur **37** relié à un picot **40** qui est entouré par un petit cylindre **41** fixé sur la face externe **42** du fond du réservoir **25** (Figure 9B).

### IV.2. Protocole.

### A. Matériel.

Pour réaliser les expériences qui suivent, on utilise des bactéries de type *Escherichia coli* qui produisent des enzymes (béta-lactamases) de résistance aux antibiotiques, CTXM et qui sont cultivées dans du milieu LB avec un antibiotique, l'ampicilline à 100 µg/ml.

### B. Méthode.

Pour cette utilisation, 1 ml d'échantillon liquide contenant les *Escherichia coli* (milieu de culture supplémenté avec de l'ampicilline 100 µg/ml) est déposé dans le préleveur. Le fond du préleveur est obturé à l'aide d'un bouchon ou un film plastique autocollant. La partie supérieure du préleveur est obturée avec un bouchon, un film plastique autocollant ou un autre système.

Le préleveur est incubé à 37°C sous agitation pendant une durée déterminée puis il est ouvert aux deux extrémités et relié au réservoir ouvert. Le bouchon/piston est vissé sur le préleveur ce qui induit la filtration de l'échantillon à travers le filtre de 0,45 µm. Les bactéries contenues dans l'échantillon restent au niveau du préleveur alors que le reste de l'échantillon (structures d'un diamètre inférieur à 0,45 µm) est éliminé via le réservoir ouvert.

Le bouchon/piston est dévissé et le réservoir ouvert est remplacé par le réservoir obturé dans lequel dans lequel ont été séchés le ou les anticorp(s) traceur(s). On dépose dans le préleveur 500 µl d'une solution permettant l'extraction des béta-lactamases bactériennes. Le bouchon piston est de nouveau vissé sur le préleveur ce qui provoque le passage de la solution d'extraction contenant les béta-lactamases dans le réservoir. La détection des béta-lactamases est réalisée en vissant le dispositif sur une cassette adaptée et dédiée à la détection de ces béta-lactamases.

### IV.3. Résultats.

Sans incubation, la limite de détection des *Escherichia coli* résistantes est de 10⁶ Unités Formant Colonies par ml (UFC/ml). Après deux heures d'incubation dans l'enceinte du dispositif i.e. après deux heures de mise en contact entre les béta-lactamases et le ou les anticorp(s) traceur(s), cette limite de détection est 10⁴ UFC/ml et après quatre heures d'incubation cette limite de détection est 10² UFC/m.

### RÉFÉRENCES

[1] Demande internationale WO 2012/118392 au nom de Infogene Lda, publiée le 7 septembre 2012.
**[2]** Demande internationale WO 2008/030817 au nom de Yong, publiée le 13 mars 2008.
**[3]** Demande internationale WO 95/08761 au nom de Polyfiltronics, Inc., publiée le 30 mars 1995.
**[4]** Demande internationale WO 2009/036168 au nom de University of Florida Research Foundation, Inc., publiée le 19 mars 2009.
**[5]** Demande internationale WO 2009/153559 au nom de The Secretary of State for Defence, publiée le 23 décembre 2009.
[6] Demande de brevet US 2016/121322 au nom de Premier Biotech, Inc., publiée le 5 mai 2016.

## Revendications

1. Dispositif (1) de prélèvement et éventuellement de traitement d'un échantillon comprenant :
une enceinte (2) avec une première extrémité (3) ouverte et une seconde extrémité (4) présentant un orifice (6),
une matrice (7) poreuse, disposée dans ladite enceinte (2) et apte à récupérer ledit échantillon,
un bouchon (11) connectable sur ladite première extrémité (3) de ladite enceinte (2) et présentant un piston (12) assurant une fermeture étanche de ladite première extrémité (3) de ladite enceinte (2), ledit bouchon (11) étant déplaçable par rapport à ladite enceinte (2) d'une position initiale à une position finale moyennant quoi ledit piston (12) compresse ladite matrice poreuse (7) ou ledit échantillon,
un réservoir (25) connectable sur ladite seconde extrémité (4) de ladite enceinte (2) et en connexion fluidique avec ladite enceinte (2) via ledit orifice (6) de façon à récupérer l'échantillon restitué par ladite matrice poreuse (7) sous l'effet de la compression de ladite matrice poreuse (7) ou dudit échantillon par ledit piston (12), ledit réservoir présentant un ou plusieurs conduit(s) (32) reliant le volume interne du réservoir à l'extérieur,
des moyens d'étanchéité (24) étant présents entre ledit bouchon (11) et ledit réservoir (25) et
lorsque ledit bouchon (11) et ledit réservoir sont connectés à ladite enceinte (2) et que ledit bouchon (11) est dans ladite position finale, ledit bouchon (11) étant en contact direct avec ledit réservoir (25) et le fermant de manière étanche et ledit ou lesdits conduit(s) (32) étant obturé(s).

2. Dispositif selon la revendication 1, **caractérisé en ce que**, lorsque ledit bouchon (11) et ledit réservoir (25) sont connectés à ladite enceinte (2) et que ledit bouchon (11) est dans la position finale, ledit bouchon (11) et ledit réservoir (25) forment un seul volume (38) fermé étanche qui contient ladite enceinte (2).

3. Dispositif selon la revendication 1 ou 2, **caractérisé en ce que** lesdits moyens d'étanchéité (24) se présentent sous forme d'un joint d'étanchéité porté par le bouchon (11).

4. Dispositif selon l'une quelconque des revendications 1 à 3, **caractérisé en ce que** ledit bouchon (11) est connectable sur ladite première extrémité (3) de ladite enceinte (2) aux moyens de filetages (9,18).

5. Dispositif selon l'une quelconque des revendications 1 à 4, **caractérisé en ce que** ladite matrice poreuse (7) est compressible.

6. Dispositif selon l'une quelconque des revendications 1 à 4, **caractérisé en ce que** ladite matrice poreuse (7) est une membrane de filtration.

7. Dispositif selon l'une quelconque des revendications 1 à 6, **caractérisé en ce que** ledit réservoir (25) présente un orifice (36) obturable par un obturateur (37) amovible.

8. Dispositif selon la revendication 7, **caractérisé en ce que** ledit dispositif présente un bouchon (46) apte à protéger ledit orifice obturable (36).

9. Dispositif selon l'une quelconque des revendications 1 à 8, **caractérisé en ce que** ledit dispositif (1) comprend en outre au moins un élément apte à traiter physiquement ou chimiquement ledit échantillon.

10. Dispositif selon la revendication 9, **caractérisé en ce que** ledit élément est une membrane de filtration.

11. Dispositif selon la revendication 9 ou 10, **caractérisé en ce que** ledit élément est un réactif chimique.

12. Système pour détecter et éventuellement quantifier un analyte d'intérêt susceptible d'être présent dans un échantillon, le système comprenant une cassette (44) présentant une ouverture (47), au moins une bandelette (52) apte à recevoir ledit échantillon via ladite ouverture (47) et présentant un indicateur visuel indiquant la présence d'un analyte d'intérêt dans ledit échantillon étant placée dans ladite cassette (44) et
un dispositif (1) selon l'une quelconque des revendications 7 à 11 en connexion fluidique avec la cassette (44) par l'intermédiaire de l'orifice (36) obturable passant d'une configuration fermée à une configuration ouverte en réponse à la connexion de ladite ouverture (47) de ladite cassette avec ledit orifice (36) obturable dudit dispositif (1).

13. Système selon la revendication 12, **caractérisé en ce que** la connexion de l'ouverture (47) de ladite cassette (44) avec l'orifice (36) obturable dudit dispositif (1) se fait par vissage.

14. Système selon la revendication 12 ou 13, **caractérisé en ce que** ladite cassette (44) comprend plusieurs bandelettes (52).

15. Kit d'éléments comprenant :
- une cassette (44) présentant une ouverture (47) éventuellement protégée par un bouchon et au moins une bandelette (52) apte à recevoir ledit échantillon via ladite ouverture (47) et présentant un indicateur visuel indiquant la présence d'un analyte d'intérêt dans ledit échantillon étant placée dans ladite cassette (44) et
- un dispositif (1) tel que défini à la revendication 8.

## Patentansprüche

1. Vorrichtung (1) zur Entnahme und gegebenenfalls Behandlung einer Probe, enthaltend:
ein Gehäuse (2) mit einem ersten offenen Ende (3) und einem zweiten Ende (4) mit einer Öffnung (6),
eine poröse Matrix (7), die in dem Gehäuse (2) angeordnet und dazu geeignet ist, die Probe aufzunehmen,
einen Stopfen (11), der mit dem ersten Ende (3) des Gehäuses (2) verbindbar ist und einen Kolben (12) aufweist, der ein dichtes Verschließen des ersten Endes (3) des Gehäuses (2) sicherstellt, wobei der Stopfen (11) relativ zu dem Gehäuse (2) von einer Anfangsposition in eine Endposition beweglich ist,
wodurch der Kolben (12) die poröse Matrix (7) oder die Probe zusammendrückt,
einen Behälter (25), der mit dem zweiten Ende (4) des Gehäuses (2) verbindbar ist und über die Öffnung (6) mit dem Gehäuse (2) in Strömungsverbindung steht, um die von der porösen Matrix (7) unter der Wirkung des Zusammendrückens der porösen Matrix (7) oder der Probe durch den Kolben (12) abgegebene Probe aufzunehmen, wobei der Behälter eine oder mehrere Leitungen (32) aufweist, die das Innenvolumen des Behälters mit dem Außenbereich verbindet bzw. verbinden,
Dichtungsmittel (24), die zwischen dem Stopfen (11) und dem Behälter (25) vorhanden sind,
wobei dann, wenn der Stopfen (11) und der Behälter mit dem Gehäuse (2) verbunden sind und sich der Stopfen (11) in der Endposition befindet, der Stopfen (11) in direktem Kontakt mit dem Behälter (25) steht und diesen dicht verschließt und die Leitung(en) (32) verschlossen ist bzw. sind.

2. Vorrichtung nach Anspruch 1,
**dadurch gekennzeichnet, dass**
dann, wenn der Stopfen (11) und der Behälter (25) mit dem Gehäuse (2) verbunden sind und sich der Stopfen (11) in der Endposition befindet, der Stopfen (11) und der Behälter (25) ein einziges, dicht verschlossenes Volumen (38) bilden, in welchem das Gehäuse (2) enthalten ist.

3. Vorrichtung nach Anspruch 1 oder 2,
**dadurch gekennzeichnet, dass**
die Dichtungsmittel (24) in Form von einer Dichtung vorliegen, die von dem Stopfen (11) getragen wird.

4. Vorrichtung nach einem der Ansprüche 1 bis 3,
**dadurch gekennzeichnet, dass**
der Stopfen (11) über Gewinde (9, 18) mit dem ersten Ende (3) des Gehäuses (2) verbindbar ist.

5. Vorrichtung nach einem der Ansprüche 1 bis 4,
**dadurch gekennzeichnet, dass**
die poröse Matrix (7) zusammendrückbar ist.

6. Vorrichtung nach einem der Ansprüche 1 bis 4,
**dadurch gekennzeichnet, dass**
die poröse Matrix (7) eine Filtermembran ist.

7. Vorrichtung nach einem der Ansprüche 1 bis 6,
**dadurch gekennzeichnet, dass**
der Behälter (25) eine Öffnung (36) aufweist, die von einem abnehmbaren Verschluss (37) verschließbar ist.

8. Vorrichtung nach Anspruch 7,
**dadurch gekennzeichnet, dass**
die Vorrichtung einen Stopfen (46) aufweist, der dazu geeignet ist, die verschließbare Öffnung (36) zu schützen.

9. Vorrichtung nach einem der Ansprüche 1 bis 8,
**dadurch gekennzeichnet, dass**
die Vorrichtung (1) ferner zumindest ein Element enthält, das dazu geeignet ist, die Probe physikalisch oder chemisch zu behandeln.

10. Vorrichtung nach Anspruch 9,
**dadurch gekennzeichnet, dass**
das Element eine Filtermembran ist.

11. Vorrichtung nach Anspruch 9 oder 10,
**dadurch gekennzeichnet, dass**
das Element ein chemisches Reagens ist.

12. System zum Nachweis und gegebenenfalls zur Quantifizierung eines interessierenden Analyten, der in einer Probe vorhanden sein kann, wobei das System enthält:
eine Kassette (44) mit einer Öffnung (47), wobei zumindest ein Teststreifen (52), der dazu geeignet ist, die Probe durch die Öffnung (47) aufzunehmen und der eine optische Anzeige aufweist, die das Vorhandensein eines interessierenden Analyten in der Probe anzeigt, in die Kassette (44) eingelegt ist, und
eine Vorrichtung (1) nach einem der Ansprüche 7 bis 11 in Strömungsverbindung mit der Kassette (44) über die verschließbare Öffnung (36), die von einer geschlossenen Konfiguration in eine offene Konfiguration als Reaktion auf die Verbindung der Öffnung (47) der Kassette mit der verschließbaren Öffnung (36) der Vorrichtung (1) übergeht.

13. System n ach Anspruch 12,
**dadurch gekennzeichnet, dass**
die Verbindung der Öffnung (47) der Kassette (44) mit der verschließbaren Öffnung (36) der Vorrichtung (1) durch Verschraubung erfolgt.

14. System nach Anspruch 12 oder 13,
**dadurch gekennzeichnet, dass**
die Kassette (44) mehrere Teststreifen (52) enthält.

15. Bausatz von Elementen, enthaltend:
- eine Kassette (44) mit einer Öffnung (47), die gegebenenfalls mittels eines Stopfens geschützt ist, und mit zumindest einem Teststreifen (52), der dazu geeignet ist, die Probe durch die Öffnung (47) aufzunehmen und eine optische Anzeige aufweist, die das Vorhandensein eines interessierenden Analyten in der Probe anzeigt, und der in die Kassette (44) eingelegt ist, und
- eine Vorrichtung (1) nach Anspruch 8.

## Claims

1. A device (1) for taking and optionally processing a sample comprising:
an enclosure (2) with a first open end (3) and a second end (4) having a hole (6),
a porous matrix (7), disposed in said enclosure (2) and able to recover said sample,
a plug (11) connectable to said first end (3) of said enclosure (2) and having a piston (12) sealingly closing said first end (3) of said enclosure (2), said plug (11) being movable with respect to said enclosure (2) from an initial position to a final position whereby said piston (12) compresses said porous matrix (7) or said sample,
a tank (25) connectable to said second end (4) of said enclosure (2) and in fluid connection with said enclosure (2) via said hole (6) so as to recover the sample restored by said porous matrix (7) under the effect of the compression of said porous matrix (7) or said sample by said piston (12), said tank having one or more conduit(s) (32) connecting the inner volume of the tank to the outside,
sealing means (24) being present between said plug (11) and said tank (25) and
when said plug (11) and said tank are connected to said enclosure (2) and when said plug (11) is in said final position, said plug (11) being in direct contact with said tank (25) and sealingly closing it and said one or more conduit(s) (32) being occluded.

2. The device according to claim 1, **characterised in that**, when said plug (11) and said tank (25) are connected to said enclosure (2) and when said plug (11) is in the final position, said plug (11) and said tank (25) form a single sealed closed volume (38) which contains said enclosure (2).

3. The device according to claim 1 or 2, **characterised in that** said sealing means (24) are in the form of a seal carried by the plug (11).

4. The device according to any of claims 1 to 3, **characterised in that** said plug (11) is connectable to said first end (3) of said enclosure (2) by means of threads (9, 18).

5. The device according to any of claims 1 to 4, **characterised in that** said porous matrix (7) is compressible.

6. The device according to any of claims 1 to 4, **characterised in that** said porous matrix (7) is a filtration membrane.

7. The device according to any of claims 1 to 6, **characterised in that** said tank (25) has a hole (36) occludable by a removable occluder (37).

8. The device according to claim 7, **characterised in that** said device has a plug (46) able to protect said occludable hole (36).

9. The device according to any of claims 1 to 8, **characterised in that** said device (1) further comprises at least one element able to physically or chemically process said sample.

10. The device according to claim 9, **characterised in that** said element is a filtration membrane.

11. The device according to claim 9 or 10, **characterised in that** said element is a chemical reagent.

12. A system for detecting and optionally quantifying an analyte of interest likely to be present in a sample, the system comprising
a cassette (44) having an aperture (47), at least one strip (52) able to receive said sample via said aperture (47) and having a visual indicator indicating the presence of an analyte of interest in said sample being placed in said cassette (44) and
a device (1) according to any of claims 7 to 11 in fluid connection with the cassette (44) through the occludable hole (36) switching from a closed configuration to an open configuration in response to the connection of said aperture (47) of said cassette with said occludable hole (36) of said device (1).

13. The system according to claim 12, **characterised in that** the connection of the aperture (47) of said cassette (44) with the occludable hole (36) of said device (1) is made by screwing.

14. The system according to claim 12 or 13, **characterised in that** said cassette (44) comprises several strips (52).

15. A kit of parts comprising:
- a cassette (44) having an aperture (47) optionally protected by a plug and at least one strip (52) able to receive said sample via said aperture (47) and having a visual indicator indicating the presence of an analyte of interest in said sample being placed in said cassette (44) and
- a device (1) as defined in claim 8.
